Europäisches Patentamt

European Patent Office

Office européen des brevets

(11) Publication number: **0 251 542**
**A2**

## EUROPEAN PATENT APPLICATION

(21) Application number: **87305265.8**

(22) Date of filing: **12.06.87**

(51) Int. Cl.⁴: **A61K 7/16**

(30) Priority: **27.06.86 JP 152541/86**

(43) Date of publication of application:
**07.01.88 Bulletin 88/01**

(84) Designated Contracting States:
**AT BE CH DE ES FR GB IT LI NL SE**

(71) Applicant: **LION CORPORATION**
**No. 3-7, 1-chome Honjyo**
**Sumida-ku Tokyo(JP)**

(72) Inventor: **Ueki, Takao**
**2-2-12, Sakurayama**
**Zushi-shi Kanagawa(JP)**
Inventor: **Suganuma, Nobuo**
**1-3-25, Yashiki**
**Narashino-shi Chiba(JP)**
Inventor: **Gomi, Tetsuo**
**3-24-7, Kouyama§Nerima-ku**
**Tokyo(JP)**

(74) Representative: **Fisher, Bernard et al**
**Raworth, Moss & Cook 36 Sydenham Road**
**Croydon Surrey CR0 2EF(GB)**

(54) **Oral composition.**

(57) An oral composition comprising a zinc salt (e.g., zinc chloride, zinc citrate, zinc acetate, zinc lactate, zinc salicylate, zinc sulfate) and 0.1% to 5% by weight of polyoxyethylene hydrogenated castor oil (e.g., having mean degree of polymerization of ethylene oxide of 10 or more). This oral composition is advantageous in that the astringency derived from the zinc salt is removed and good overall sensation in the mouth is obtained.

EP 0 251 542 A2

## ORAL COMPOSITION

BACKGROUND OF THE INVENTION

I. Field of the Invention

The present invention relates to an oral composition having an oral malodor relieving effect by formulating a zinc salt. More specifically, it relates to an oral composition containing a zinc salt in which the astringency caused by the formulation of the zinc salt can be improved and a good overall sensation in the mouth is provided.

2. Description of the Related Art

It has heretofore been known in the art that zinc salts such as zinc chloride are effective as an oral malodor relieving agent or oral cleaning agent. For example, JP-A-60-233008 discloses the formulation of zinc salts into oral compositions such as mouth washes and dentifrices.

However, when zinc salts such as zinc chlorides are formulated into oral compositions, the astringency of the oral compositions becomes strong and the desired flavor is lost, and as a result, the overall sensation in the mouth is disadvantageously impaired.

SUMMARY OF THE INVENTION

Accordingly, the objects of the present invention are to eliminate the above-mentioned disadvantages of the prior art and to provide an oral composition capable of effectively removing the undesirable astringency caused by the formulation of a zinc salt therein and of affording a good overall sensation in the mouth.

Other objects and advantages of the present invention will be apparent from the description set forth hereinbelow.

In accordance with the present invention, there is provided an oral composition comprising a zinc salt and 0.1% to 5% by weight of polyoxyethylene hydrogenated castor oil.

DESCRIPTION OF THE PREFERRED EMBODIMENT

The present inventors carried out an extensive study of ways in which to achieve the above-mentioned objects of the present invention and, as a result, found that, when polyoxyethylene hydrogenated castor oil is formulated into an oral composition containing a zinc salt, the astringency caused by the formulation of a zinc salt into an oral composition can be unexpectedly removed and an oral composition capable of providing a good overall sensation in the mouth can be obtained. As mentioned above, an oral composition containing a zinc salt is disclosed in JP-A-60-233008, but although the use of polyoxyethylene polyoxypropylene block copolymer type nonionic surfactant (i.e., Pluronic F-85) is disclosed in the examples of this Japanese patent reference, such Pluronic type surfactants cannot remove the astringency derived from a zinc salt, even used in combination with the zinc salt. Furthermore, although polyoxyethylene sorbitan fatty acid esters and polyoxyethylene alkyl ethers are polyoxyethylene type surfactant, they improve the astringency concerned. Nevertheless, when polyoxyethylene hydrogenated castor oil is used in combination with a zinc salt, it has been unexpectedly found that the astringency derived from the zinc salt can be removed and the overall sensation in the mouth, when such an oral composition is used, can be improved. When the polyoxyethylene hydrogenated castor oil is formulated in a large amount, oiliness derived from the polyoxyethylene hydrogenated castor oil is caused. Accordingly, the polyoxyethylene hydrogenated castor oil is used, in the present oral composition containing a zinc salt, in an amount of 0.1% to 5% by weight, based on the total amount of the composition.

As mentioned above, the present oral composition contains, as an essential constituent, a zinc salt, whereby the desired oral malodor relieving effect can be provided. Although there are no limitations to the zinc salts usable in the present invention, preferable examples of these zinc salts are zinc chloride, zinc citrate, zinc acetate, zinc lactate, zinc salicylate, and zinc sulfate. These zinc salts may be used alone or in any mixtures thereof.

Although there are no critical limitations to the amount of the zinc salt to be formulated, the amount of the zinc salt in the present oral composition is preferably 0.01% to 1% by weight, more preferably 0.02% to 0.5% by weight, in terms of a zinc ion, based on the total amount of the composition. When the amount of the zinc ion is less than 0.01% by weight, the desired effect obtained by the formulation of the zinc salt is not obtained. Conversely, when the amount of the zinc ion is more than 1% by weight, the flavor of the oral composition or the overall sensation in mouth when used is sometimes remarkably impaired.

As mentioned above, according to the present invention, polyoxyethylene hydrogenated castor oil is formulated into the zinc-containing oral composition to improve the astringency derived from the zinc salt. The preferable polyoxyethylene hydrogenated castor oil includes those having an ethylene oxide addition mole number of 10 mole or more from the viewpoint of low temperature stability. Especially, polyoxyethylene hydrogenated castor oil having an ethylene oxide addition mole number of 20 to 100 moles can be advantageously used in the present invention, since the low temperature stability thereof is excellent even when compared with, for example, Pluronic type surfactants. Accordingly, when a transparent oral composition such as a mouth wash is prepared, turbidity is not generated in the oral composition when stored at a low temperature and, therefore, the polyoxyethylene hydrogenated castor oil can be preferably and advantageously used in the preparation of the above-mentioned transparent oral compositions.

The amount of the polyoxyethylene hydrogenated castor oil to be formulated should be 0.1% to 5% by weight, preferably 0.3% to 4% by weight, based on the total amount of the oral composition. When the amount of the polyoxyethylene hydrogenated castor oil is less than 0.1% by weight, the astringency derived from the zinc salt cannot be sufficiently reduced. Conversely, when the amount of the polyoxyethylene hydrogenated castor oil is more than 5% by weight, the oiliness of polyoxyethylene hydrogenated castor oil is unpreferably caused.

The present oral composition may contain, in addition to the above-mentioned essential components *l*-menthol. When *l*-menthol is formulated into the oral composition, in addition to the above-mentioned zinc salt and polyoxyethylene hydrogenated castor oil, the taste mildness of the oral composition is advantageously improved and the overall sensation in the mouth is further improved. When *l*-menthol is formulated into the present oral composition, the amount of *l*-menthol formulated therein is preferably 0.01% to 3% by weight, more preferably 0.05% to 2% by weight, based on the total amount of the oral composition. When the amount of *l*-menthol is less than 0.1% by weight, the intended effect derived from *l*-menthol is not obtained. Conversely, when the amount of *l*-menthol is more than 3% by weight, a bitter taste derived from the *l*-menthol per se is sometimes felt.

The oral compositions according to the present invention can be formulated as, for example, tooth paste, wet dentifrice, liquid dentifrice, mouth wash, oral pasta, and spray type breath freshener.

The present oral composition may optionally contain any conventional ingredients depending upon the kind of oral composition and the intended use thereof. Examples of such ingredients are abrasives such as calcium phosphate (dibasic)•dihydrate, calcium phosphate (dibasic)•anhyd, calcium phosphate (monobasic), calcium phosphate (tribasic), calcium carbonate, calcium pyrophosphate, insoluble sodium metaphosphate, amorphous silica, crystalline silica, aluminosilicate, aluminum hydroxide, aluminum oxide, magnesium phosphate (tribasic), magnesium carbonate, magnesium sulfate, titanium oxide, and resins; binders such as sodium carboxymethyl cellulose, methyl cellulose, sodium carboxymethylhydroxyethyl cellulose, hydroxyethyl cellulose, sodium alginate, carageenan, gum arabic, xanthan gum, tragacanth gum, gum karaya, polyvinyl alcohol, sodium polyacrylate, carboxyvinyl polymer, and polyvinyl pyrrolidone; humectants such as polyethylene glycol, ethylene glycol, sorbitol, glycerol, propylene glycol, 1,3-butylene glycol, xylite, maltite, and lactite; sweeteners such as saccharin sodium, stebiocide, neohesperidyl dihydrochalcon, glycyrrhizn, perillartine, and p-methoxy cinnamic aldehyde; and preservatives such as benzoic acid, sodium benzoate, methyl parahydroxy benzoate, ethyl parahydroxybenzoate, propyl parahydroxybenzoate, butyl parahydroxy benzoate, sodium dehydroacetate, potassium sorbate, and sodium propionate. Furthermore, the present oral composition may further contain other surfactants in addition to the polyoxyethylene hydrogenated castor oil, other flavors in addition to *l*-menthol, and other active ingreadients in addition to the zinc salt.

The oral compositions according to the present invention may be prepared in any conventional manner by, for example, mixing the above-mentioned essential and optional ingredients with water.

As mentioned above, according to the present invention, the oral composition in which the astringency of the zinc salt is reduced, and providing a good overall sensation in the mouth, can be obtained by using the polyoxyethylene hydrogenated castor oil in combination with the zinc salt.

EXAMPLES

The present invention will now be further illustrated by, but is by no means limited to, the following Experiments and Examples, wherein "%" means "% by weight" unless otherwise specified.

Experiment I

The mouth wash sample Nos. I to I3 having the following formulations including the surfactants listed in Table I were prepared.

## Formulation of Mouth Wash

| Ingredient | % |
|---|---|
| Ethyl alcohol | 10.0 |
| Glycerol | 10.0 |
| Citric acid | 0.1 |
| Sodium citrate | 0.4 |
| Sodium benzoate | 0.3 |
| Sodium lauryl sulfate | 0.5 |
| Flavor | 0.8 |
| Saccharin sodium | 0.05 |
| FD & C Green #3 (dye) | 0.001 |
| D & C Yellow #10 | 0.001 |
| Zinc chloride | 0.1 |
| Surfactant | Amount shown in Table 1 |
| Purified water | balance |
| Total | 100.0% |

The feeling upon application (i.e., astringency, oiliness, and overall flavor) of the mouth wash that samples was organoleptically evaluated, on average, by 10 expert evaluators under the following standars. The results are shown in Table I.

4

## Evaluation Standards

| Score | Astringency | Oiliness | Overall Flavor |
|-------|-------------|----------|----------------|
| 4 | No astringency | No oiliness | Very good flavor |
| 3 | Weak astringency | Substantially no oiliness | Good flavor |
| 2 | Rather strong astringency | Some oiliness | Fair flavor |
| 1 | Strong astringency | Oiliness | Poor flavor |

## Evaluation

| Average of 10 members | Evaluation |
|-----------------------|------------|
| 3.0 - 4.0 | o |
| 2.0 - 2.9 | Δ |
| 1.0 - 1.9 | x |

Table 1

| Sample No.<br>Component | No. 1 | No. 2 | No. 3 | No. 4 | No. 5 | No. 6 | No. 7 | No. 8 | No. 9 | No. 10 | No. 11 | No. 12 | No. 13 |
|---|---|---|---|---|---|---|---|---|---|---|---|---|---|
| POE (40) hydroge-<br>nated castor oil | – | 0.05 | 0.10 | 1.00 | 2.00 | 5.00 | 10.00 | – | – | – | – | – | – |
| Pluronic F-127 | – | – | – | – | – | – | – | 2.00 | – | – | – | – | – |
| Pluronic P-85 | – | – | – | – | – | – | – | – | 2.00 | – | – | – | – |
| Pluronic F-68 | – | – | – | – | – | – | – | – | – | 2.00 | – | – | – |
| POE (20) sorbitan monooleate | – | – | – | – | – | – | – | – | – | – | 2.00 | – | – |
| POE (20) sorbitan monooleate | – | – | – | – | – | – | – | – | – | – | – | 2.00 | – |
| POE (9) lauryl ether | – | – | – | – | – | – | – | – | – | – | – | – | 2.00 |
| Astringency | x | x | Δ | o | o | o | o | x | x | x | x | x | x |
| Oiliness | o | o | o | o | o | Δ | x | o | o | o | x | x | x |
| Overall flavor | x | x | Δ | o | o | Δ | x | x | x | x | x | x | x |

(Note) The "Pluronic T-127, P-85, and F-68" components are all available from Wyandotte Chemical Co. and have the following compositions.

$$HO \overbrace{( POE )}_{a} ( POP )_{b} ( POE )_{c} H$$

|       | a  | b  | c  |
|-------|----|----|----|
| F-127 | 98 | 67 | 98 |
| P-85  | 27 | 39 | 27 |
| F-68  | 75 | 30 | 75 |

As is clear from the results shown in Table I, the astringency of the mouth wash containing a zinc salt can be removed by formulating 0.1% to 5% of polyoxyethylene hydrogenated castor oil therein.

7

Experiment 2

The mouth wash sample Nos. 14 to 21 having the formulations below were prepared by using zinc chloride in the amounts listed in Table 2 below.

## Formulation of Mouth Wash

| Ingredient | % |
| --- | --- |
| Ethyl alcohol | 10.0 |
| Glycerol | 10.0 |
| Citric acid | 0.1 |
| Sodium citrate | 0.4 |
| Sodium benzoate | 0.3 |
| Sodium lauryl sulfate | 0.5 |
| Flavor | 0.8 |
| Sodium saccharinate | 0.05 |
| FD & C Green #3 | 0.001 |
| D & C Yellow #10 | 0.001 |
| POE (40) hydrogenated castor oil | 2.0 |
| Zinc chloride | Amount shown in Table 1 |
| Purified water | balance |
| Total | 100.0% |

The oral malodor relieving effects of the above-prepared samples were evaluated in the following manner and by the following standard. The overall sensation in mouth (flavor) of these mouth washes was also evaluated in the same manner as in Experiment I.

The results are shown in Table 2.

Evaluation of Oral Malodor Relieving Effect Method

The oral malodor of 10 panelists was evaluated before mouth washing, in accordance with the following standard, by three expert evaluaters. The average evaluation value was calculated.

The panelists then washed their mouths with 30 ml of the test samples, and after 15 minutes, the oral malodor of the panelists was evaluated in the same manner as the evaluation before the mouth washing, by the three expert evaluators.

The intervals of the mouth washing test of the same panelist was at least 3 hours.

## Evaluation Standard

| Score | Level of Oral Malodor |
|-------|----------------------|
| 4 | Strong |
| 3 | Fair |
| 2 | Slight |
| 1 | None |

Evaluation

The oral malodor relieving effects were determined based on the differences between the evaluation averages before and after the mouth washing.

| Difference between evaluation average before and after mouth washing | Evaluation |
|---|---|
| 2.0 - 3.0 | O |
| 1.0 - 1.9 | Δ |
| 0 - 0.9 | x |

Table 2

| Sample Ingredient | No. 14 | No. 15 | No. 16 | No. 17 | No. 18 | No. 19 | No. 20 | No. 21 |
|---|---|---|---|---|---|---|---|---|
| Zinc chloride (as Zn ion) | — | 0.01 (0.005) | 0.02 (0.01) | 0.1 (0.05) | 0.2 (0.1) | 1.0 (0.5) | 2.0 (1.0) | 4.0 (2.0) |
| Oral malodor relieving effect | x | x | △ | ○ | ○ | ○ | ○ | ○ |
| Flavor | ○ | ○ | ○ | ○ | ○ | ○ | △ | x |

As is clear from the results shown in Table 2, the effective amount, in terms of Zn ion, of the zinc salt to be formulated into the oral composition is preferably 0.01% or more from the viewpoint of the oral malodor relieving effect and preferably 1% or less from the viewpoint of the flavor.

Experiment 3

The mouth wash sample Nos. 22 to 28 containing $l$-menthol in the amounts listed in Table 3 and having the following formulations were prepared.

## Formulation of Mouth Wash

| Ingredient | % |
| --- | --- |
| Ethyl alcohol | 10.0 |
| Glycerol | 10.0 |
| Citric acid | 0.1 |
| Sodium citrate | 0.4 |
| Sodium benzoate | 0.3 |
| Sodium lauryl sulfate | 0.5 |
| Flavor other than $l$-menthol | 0.5 |
| Sodium saccharinate | 0.05 |
| FD & C Green #3 | 0.001 |
| D & C Yellow #10 | 0.001 |
| Zinc chloride | 0.1 |
| POE (40) hydrogenated castor oil | 2.0 |
| $l$-Menthol | Amount listed in Table 3 |
| Purified water | balance |
| Total | 100.0% |

The feeling upon application (i.e., mildness of taste, bitter taste, and astringency) of the mouth wash test samples were evaluated by 10 expert evaluators under the following standards. The evaluation was made by taking the average score of the 10 members.

11

## Evaluation Standards

| Score | Taste Mildness | Astringency |
|-------|----------------|-------------|
| 4 | Excellent | No bitter taste |
| 3 | Good | Slight bitter taste |
| 2 | Fair | Some bitter taste |
| 1 | Poor | Substantial bitter taste |

| Average of 10 members | Evaluation |
|-----------------------|------------|
| 3.0 - 4.0 | o |
| 2.0 - 2.9 | Δ |
| 1.0 - 1.9 | x |

The astringency was evaluated in the same manner as in Experiment I.
The results are shown in Table 3.

12

Table 3

| Sample<br>Ingredient | No. 22 | No. 23 | No. 24 | No. 25 | No. 26 | No. 27 | No. 28 |
|---|---|---|---|---|---|---|---|
| *l*-Menthol | — | 0.01 | 0.1 | 0.5 | 1.0 | 3.0 | 5.0 |
| Mildness of taste | x | △ | ○ | ○ | ○ | ○ | ○ |
| Bitter taste | ○ | ○ | ○ | ○ | ○ | △ | x |
| Astringency | ○ | ○ | ○ | ○ | ○ | ○ | ○ |

As is clear from the results shown in Table 3, a taste mildness can be obtained when *l*-menthol is formulated into the mouth wash containing the zinc salt, but the use of too large an amount of *l*-menthol in the mouth wash causes a bitter taste. Accordingly, the preferable amount of the *l*-menthol to be formulated into the oral composition is 0.01% to 3%.

13

Experiment 4

The mouth wash sample Nos. 29 to 37 having the following compositions were prepared by using the surfactants listed in Table 4.

## Formulation of Mouth Wash

| Ingredient | % |
|---|---|
| Ethyl alcohol | 10.0 |
| Glycerol | 10.0 |
| Citric acid | 0.1 |
| Sodium citrate | 0.4 |
| Sodium benzoate | 0.3 |
| Sodium lauryl sulfate | 0.5 |
| Flavor | 0.8 |
| Sodium saccharinate | 0.05 |
| FD & C Green #3 | 0.001 |
| D & C Yellow #10 | 0.001 |
| Zinc chloride | 0.1 |
| Surfactant | 2.0 |
| Purified water | balance |

100.0%

The low temperature stability of the samples was evaluated as follows.

14

Evaluation Method

The samples were stored at a temperature of -10°C for one week and the transparency of the solution was evaluated at a temperature of -10°C.

## Evaluation

| Transparency | Evaluation |
|---|---|
| Transparent | o |
| Little turbidity | Δ |
| Turbid | x |

The results are shown in Table 4.

## Table 4

| Sample | Ingredient | Low temperature stability |
|---|---|---|
| No. 29 | POE (5) hydrogenated castor oil | x |
| No. 30 | POE (10) hydrogenated castor oil | Δ |
| No. 31 | POE (20) hydrogenated castor oil | o |
| No. 32 | POE (40) hydrogenated castor oil | o |
| No. 33 | POE (60) hydrogenated castor oil | o |
| No. 34 | POE (100) hydrogenated castor oil | o |
| No. 35 | Pluronic F-127 | x |
| No. 36 | Pluronic P-85 | x |
| No. 37 | Pluronic F-68 | x |

As is clear from the results shown in Table 4, the use of the POE (i.e., polyoxyethylene) hydrogenated castor oil having a mean degree of polymerization of ethylene oxide of 10 to 100 is preferable from the viewpoint of low temperature stability.

The present invention will now be further illustrated by the following Examples.

Examples 1 to 6: Mouth Wash

| Ingredient | Example No. 1 | 2 | 3 | 4 | 5 | 6 |
|---|---|---|---|---|---|---|
| Ethyl alcohol | 10.0 | 15.0 | 20.0 | 5.0 | 10.0 | 15.0 |
| Glycerol | 10.0 | - | 5.0 | 15.0 | 10.0 | 10.0 |
| Sorbitol | - | 10.0 | 5.0 | - | - | - |
| Citric acid | 0.1 | 0.05 | 0.1 | 0.05 | 0.1 | 0.05 |
| Sodium citrate | 0.4 | 0.2 | 0.4 | 0.2 | 0.4 | 0.2 |
| Sodium benzoate | 0.3 | 0.2 | 0.1 | - | - | - |
| Methyl paraben | - | - | - | 0.1 | 0.05 | - |
| Sodium lauryl sulfate | 0.5 | 0.3 | 0.1 | - | 0.5 | 0.3 |
| Flavor (other than ℓ-menthol) | 0.5 | 0.3 | 0.8 | 0.8 | 0.5 | 0.8 |
| Saccharin sodium | 0.05 | 0.05 | 0.05 | 0.05 | 0.05 | 0.05 |
| FD & C Green #3 | 0.001 | - | 0.001 | - | 0.001 | - |
| FD & C Blue #1 | - | 0.001 | - | 0.001 | - | 0.001 |
| FD & C Yellow #5 | - | 0.001 | 0.001 | - | - | 0.001 |
| D & C Yellow #10 | 0.001 | - | - | 0.001 | 0.001 | - |
| Zinc chloride | 0.1 | - | - | - | - | - |
| Zinc citrate | - | 0.2 | - | - | - | - |
| Zinc acetate | - | - | 0.2 | - | - | - |
| Zinc lactate | - | - | - | 0.5 | - | - |
| Zinc salicylate | - | - | - | - | 0.5 | - |
| Zinc sulfate | - | - | - | - | - | 0.2 |
| POE (20) hydrogenated castor oil | - | 3.0 | - | - | - | - |
| POE (40) hydrogenated castor oil | 2.0 | - | - | - | - | 1.0 |
| POE (60) hydrogenated castor oil | - | - | 1.0 | - | 2.0 | - |
| POE (80) hydrogenated castor oil | - | - | - | 2.0 | - | - |
| ℓ-Menthol | 0.3 | 0.8 | 0.1 | - | 0.3 | 0.1 |
| Purified water | balance | balance | balance | balance | balance | balance |
| Total | 100.0 | 100.0 | 100.0 | 100.0 | 100.0 | 100.0 |

Examples 7 to 12:  Toothpaste

| Example No.<br>Ingredient | 7 | 8 | 9 | 10 | 11 | 12 (%) |
|---|---|---|---|---|---|---|
| Calcium phosphate (dibasic) | 50 | - | - | - | - | 40 |
| Aluminum hydroxide | - | 40 | - | - | - | - |
| Calcium carbonate | - | - | 40 | - | - | - |
| Zircono silicate | - | - | - | 30 | - | - |
| Silicic anhydride | - | - | - | 2 | 25 | 2 |
| Sodium carboxymethyl cellulose | 0.5 | - | 1.0 | 1.0 | 1.0 | 1.0 |
| Carageenan | 0.5 | 0.5 | - | - | - | - |
| Sodium alginate | - | 0.5 | - | - | - | - |
| Glycerol | 10 | - | - | - | - | 15 |
| Sorbitol | 10 | 30 | 20 | 30 | 30 | - |
| Propylene glycol | 2.0 | 2.0 | 2.0 | - | - | - |
| PEG #400 | - | - | - | 5.0 | 5.0 | 3.0 |
| Ethyl alcohol | - | - | 0.5 | - | - | - |
| Sodium lauryl sulfate | 1.5 | 1.5 | 1.5 | 1.5 | 1.5 | 1.0 |
| Sodium lauroyl sarcosinate | - | - | - | - | 0.3 | 0.3 |
| Lauric acid diethanol amide | - | 0.3 | - | 0.3 | 0.3 | - |
| Sodium benzoate | 0.1 | 0.1 | 0.5 | - | - | - |
| Methyl paraben | 0.1 | 0.1 | - | 0.1 | 0.1 | 0.2 |
| Butyl paraben | - | - | - | 0.1 | 0.1 | 0.1 |
| Saccharin sodium | 0.1 | 0.1 | 0.1 | 0.1 | 0.1 | 0.1 |
| FD & C Blue #1 | - | - | - | 0.001 | 0.001 | - |
| Flavor (other than ℓ-menthol) | 0.7 | 1.0 | 0.5 | 0.8 | 0.3 | 0.7 |
| Sodium monofluorophosphate | - | 0.76 | - | - | - | - |
| Dextranase (1000000 U/g) | - | 0.2 | - | - | - | - |
| Sodium chloride | - | - | 10.0 | - | - | - |
| Tranexamic acid | - | - | 0.1 | - | - | 0.1 |
| Tocopherol acetate | - | - | - | - | - | 0.1 |
| β-Glycyrrhetinic acid | - | - | - | - | - | 0.05 |
| Chlorohexidine hydrochloride | - | - | - | 0.01 | - | 0.02 |
| Zinc chloride | 0.2 | 0.3 | 0.1 | - | - | - |
| Zinc citrate | - | - | - | 0.2 | 0.3 | 0.1 |
| POE (20) hydrogenated castor oil | 0.5 | - | 0.3 | - | 2.0 | - |
| POE (40) hydrogenated castor oil | - | 0.5 | - | 1.0 | - | 2.0 |
| ℓ-Menthol | 0.2 | - | 0.5 | 0.1 | 0.8 | 0.3 |
| Purified water | balance | balance | balance | balance | balance | balance |
| Total | 100.0 | 100.0 | 100.0 | 100.0 | 100.0 | 100.0 |

Example 13:  Wet Dentifrice

| Ingredient | % |
| --- | --- |
| Calcium carbonate | 80.0 |
| Silicic anhydride | 2.0 |
| PEG #4000 | 1.0 |
| PEG #400 | 10.0 |
| Sodium lauryl sulfate | 1.5 |
| Saccharin sodium | 0.1 |
| Flavor other than ℓ-Menthol | 0.8 |
| Zinc chloride | 0.1 |
| POE (20) hydrogenated castor oil | 1.0 |
| ℓ-Menthol | 0.2 |
| Purified water | balance |
| Total | 100.0% |

Example 14: Liquid Dentifrice

| Ingredient | % |
| --- | --- |
| Glycerol | 35.0 |
| Propylene glycol | 5.0 |
| Sodium polyacrylate | 2.0 |
| Sodium lauryl sulfate | 1.0 |
| Lauric acid diethanolamide | 1.5 |
| Saccharin sodium | 0.2 |
| Sodium benzoate | 0.3 |
| Flavor other than l-menthol | 0.5 |
| Zinc chloride | 0.2 |
| POE (60) hydrogenated castor oil | 2.0 |
| l-Menthol | 0.5 |
| Purified water | balance |
| Total | 100.0% |

19

### Example 15:  Oral Pasta

| Ingredient | % |
| --- | --- |
| Liquid paraffin | 25.0 |
| Sorbitol | 15.0 |
| Cetanol | 5.0 |
| Paraffin wax | 5.0 |
| Microcrystalline wax | 10.0 |
| Methyl paraben | 0.2 |
| Sodium benzoate | 0.1 |
| Flavor other than ℓ-menthol | 0.5 |
| Zinc chloride | 0.1 |
| POE (40) hydrogenated castor oil | 4.0 |
| ℓ-Menthol | 1.0 |
| Purified water | balance |
| Total | 100.0% |

### Example 16:  Spray Type Breath Freshener

| Ingredient | % |
| --- | --- |
| Ethyl alcohol | 50.0 |
| Glycerol | 10.0 |
| Saccharin sodium | 0.3 |
| Flavor other than ℓ-menthol | 0.7 |
| Zinc chloride | 0.1 |
| POE (60) hydrogenated castor oil | 3.0 |
| ℓ-Menthol | 0.3 |
| Purified water | balance |
| Total | 100.0% |

The oral compositions of Examples I to I6 all gave a good feeling upon application (i.e., flavor) and had good oral malodor relieving effects.

## Claims

I. An oral composition comprising a zinc salt and 0.I% to 5% by weight of polyoxyethylene hydrogenated castor oil.

2. An oral composition as claimed in claim I, wherein the amount of the zinc salt is 0.0I% to I% by weight, in terms of a zinc ion, based on the total weight of the oral composition.

3. An oral composition as claimed in claim I, wherein said zinc salt is at least one compound selected from the group consisting of zinc chloride, zinc citrate, zinc acetate, zinc lactate, zinc salicylate, and zinc sulfate.

4. An oral composition as claimed in claim I, wherein said polyoxyethylene hydrogenated castor oil has mean degree of polymerization of ethylene oxide of I0 or more.

5. An oral composition as claimed in claim I, wherein said composition further comprises 0.0I% to 3% by weight of $l$-menthol.